(19) Europäisches Patentamt
European Patent Office
Office européen des brevets

(11) Numéro de publication: 0 246 946
A2

# (12) DEMANDE DE BREVET EUROPEEN

(21) Numéro de dépôt: 87401014.3

(51) Int. Cl.4: A 61 K 31/565

(22) Date de dépôt: 04.05.87

(30) Priorité: 05.05.86 FR 8606457

(43) Date de publication de la demande:
25.11.87 Bulletin 87/48

(84) Etats contractants désignés:
AT BE CH DE ES FR GB GR IT LI LU NL SE

(71) Demandeur: Laboratoire Théramex
2 Boulevard Charles III
Monaco (MC)

(72) Inventeur: Paris, Jacques
Le Clos de Cimiez E1 31 Cap De Croix
F-06100 Nice (FR)

(74) Mandataire: Burtin, Jean-François
Cabinet GEFIB 6, Place Boulnois
F-75017 Paris (FR)

Revendications pour les Etats contractants suivants: ES + GR.

(54) **Nouvelles compostions pharmaceutiques à action anti-progestéronique et leur procédé d'obtention.**

(57) La présente invention a pour objet de nouvelles compositions pharmaceutiques à action anti-progestéronique .

Elle se caractérise par le fait que ces compositions renferment à titre de principe actif une quantité physiologiquement-active de 17β-OR 17α-ethynyl 5α-adrost-2-ène en association ou en mélange avec un excipient ou un vehicule pharmaceutique.

Ces compositions pharmaceutiques servent comme médicaments anti-progestérone,notamment pour empêcher la nidation ou la gestation.

EP 0 246 946 A2

## Description

### NOUVELLES COMPOSITIONS PHARMACEUTIQUES A ACTION ANTIPROGESTERONIQUE ET LEUR PROCEDE D'OBTENTION

La présente invention a pour objet de nouvelles compositions pharmaceutiques qui inhibent les actions de la progestérone.

Elle a plus particulièrement pour objet de nouvelles compositions pharmaceutiques caractérisées en ce qu'elles renferment une quantité antiprogestéronique d'un composé androst-2-énique de formule générale I:

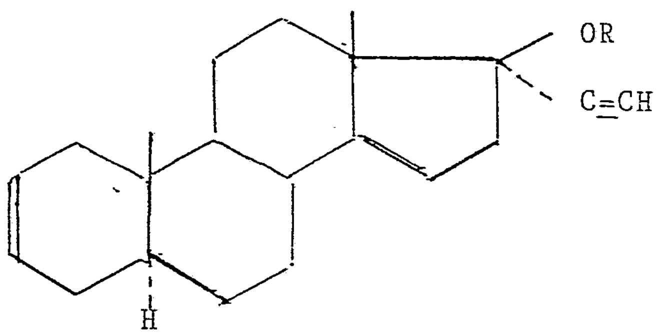

dans laquelle:
R est un hydrogène ou le reste acyle d'un acide organique carboxylique, un radical alcoyle ou tétrahydropyranyle,
en association ou en mélange avec un excipient ou un véhicule inerte non toxique pharmaceutiquement acceptable.

Ces composés, et plus particulièrement le 17-acétate, ont déjà été décrits dans la littérature, notamment dans le brevet britannique 1 492 746. Plus récemment, des procédés de purification ont été décrits comme par exemple dans le brevet européen 66 601 ou dans la demande de brevet européen 86 4006226 déposée le 25 mars 1986 au nom de la société demanderesse. Leurs propriétés biologiques connues se caractérisent par des effets de freination gonadotrope hypophysaire et gonadique, se manifestant par l'involution de l'ovaire et du testicule, chez l'animal de Laboratoire.

Les applications thérapeutiques déjà divulguées ont concerné l'endométriose, les maladies bénignes du sein et la pseudogestation chez l'animal, la chienne notamment.

Des études biologiques récentes ont révélé que ces composés, utilisés par voie orale ou injectable, dans les conditions décrites ci-après, sont susceptibles d'interférer puissamment avec les effets de la progestérone, se comportant comme un antagoniste de cette hormone.

Même à une forte dose (25 mg chez la lapine), les composés de formule générale I, dont le principal représentant l'acétate de 17α-éthynyl-17β-hydroxy(5α)androstène-2, n'entraînent qu'un effet pseudogestatif faible (< 2,5% de la progestérone). A l'inverse, ils antagonisent l'effet de la progestérone après injection, aussi bien par voie orale que parentérale, chez la lapine. Cette évaluation de l'effet inhibiteur de la prolifération endométriale, par la progestérone, est basée sur la détermination selon le test de Clauberg et de l'indice de Mac Phail).

L'inhibition est significative après administration par voie sous-cutanée, à la dose de 5 mg et totale à la dose de 10 mg vis-à-vis de l'effet de 0,24 mg de progestérone injectable; elle est aussi très importante à la dose de 20 mg vis-à-vis de 1,9 mg de progestérone injectable.

Par voie orale, l'effet du 17-acétate de 17α-éthynyl-17β-hydroxy-5α-androstène-2 17β-ol est plus important: l'inhibition est pratiquement totale à 5 mg vis-à-vis de 0,24 mg de progestérone injectable; elle est très marquée à la dose de 10 mg vis-à-vis de 1,9 mg de progestérone injectable.

Administrés par voie orale avant la nidation (jour 1 à 5 après fécondation chez la ratte gestante, à la dose de 5 mg/kg/j), les composés de formule générale I rendent la gestation impossible. Dans les mêmes conditions de traitement, ils diminuent le nombre des implantations de foetus, dès la dose de 1,25 mg/kg/j chez la ratte.

Chez la lapine, la gestation n'est plus possible dès la dose de 5 mg/kg/j, administrée à partir du moment de la fécondation.

Le 17β-acétate du 17α-éthynyl(5α)androstène-2 17β-ol possède donc également un effet anti-nidatoire et un effet abortif.

Les compositions pharmaceutiques selon l'invention constituent un médicament efficace pour empêcher la nidation et la gestation humaine, s'il est utilisé au début de la grossesse. Il peut être utilisé par voie orale ou parentérale.

Les doses nécessaires se situent entre 50 et 200 mg/jour, pendant plusieurs jours et l'administration pour 24 heures peut être fractionnée en plusieurs prises.

Une autre application clinique des compositions selon l'invention, conséquence de l'effet antiprogestatif, est l'induction volontaire des menstruations avant la fin de la phase lutéale d'un cycle normal.

Cet effet antiprogestéronique qui entraîne une action antiprogestative est surprenant car il n'est la conséquence ni de l'activité antigonadotrope, ni d'un effet de compétition au niveau moléculaire car les composés de formule générale I ne manifestent par d'affinité pour les récepteurs spécifiques de la progestérone, au contraire des effets constatés avec le composé RU 31486.

Les compositions pharmaceutiques selon l'invention contiennent donc de 25 à 250 mg d'un composé de formule générale I par prise unitaire.

Les formes pharmaceutiques selon l'invention sont celles qui conviennent pour l'administration par voie parentérale,orale,rectale,vaginale.

A cette fin, elles sont présentées sous forme de comprimés nus ou enrobés, de dragées, de capsules, de tablettes, de comprimés à enrobage protecteur, de capsules molles, de gélules, de pilules, de cachets, de solutions ou suspensions buvables, de solutés injectables répartis en ampoules, en seringues auto-injectables ou en flacons multi-doses.

Parmi les véhicules ou les excipients, on pourra citer plus particulièrement ceux qui conviennent pour l'administration par voie parentérale ou par voie digestive. On pourra citer à cet égard l'eau, les solutés de chlorure de sodium, les solutions isotoniques, les solutés de polyéthylène glycol dans l'eau, les solutions aqueuses de polyvinylpyrrolidone pour les formes injectables, les diluants usuels comme les amidons, les celluloses, le carbonate de magnésium, le phosphate de calcium, la silice, le stéarate de magnésium, le talc; les agents liants, les agents édulcorants, parfumants ou de sapidité pour les formes solides. On peut également utiliser des formed appropriées pour l'administration par voie rectale comme les suppositoires ou les capsules rectales. Les excipients utilisés seront le beurre de cacao ou les stéarates de polyéthylèneglycol.

L'invention comprend aussi un procédé d'obtention des compositions pharmaceutiques à action antiprogestéronique caractérisé en ce qu'on mélange ou ajoute une quantité antiprogestéronique d'un composé de formule générale I:

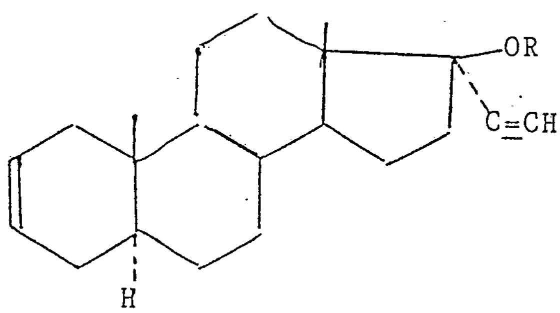

dans laquelle R a les définitions fournies précédemment,
à un excipient ou à un véhicule inerte non toxique pharmaceutiquement acceptable.

Cette incorporation ou ce mélange s'effectue selon les méthodes usuelles de la pharmacotechnie.

La partie expérimentale qui figure ci-après fournit un résumé des essais pratiqués sur l'animal et illustre l'invention.

EXEMPLE I

Comprimés de 17α-éthynyl-17β-acétoxy-5α-androst-2-ène:

| | |
|---|---|
| . 17α-éthynyl-17β-acétoxy-5α-androst-2-ème .. | 50 g |
| . amidon de blé ............................ | 47 g |
| . amidon de maïs ........................... | 61 g |
| . cellulose microcristalline ................ | 12 g |
| . polyvinylpyrrolidone pontée ............... | 8 g |
| . carbonate de magnésium .................... | 26 g |
| . stéarate de magnésium ..................... | 15 g |
| . talc ...................................... | 6 g |

pour mille comprimés terminés au poids moyen de 225 mg.

EXEMPLE II

Soluté injectable de 17α-éthynyl-17β-acétoxy-5α-androst-2-ène:

- 17α-éthynyl-17β-acétoxy-5α-androst-2-ène .. 10 g
- alcool benzylique ........................ 4,5 ml
- huile d'olive stérile ................ q.s.p. 200 ml

Le soluté est réparti en ampoules de 2 ml contenant chacune 100 mg de principe actif.

EXEMPLE III

Effet progestomimétique et antiprogestéronique des composés de formule I:

L'action antiprogestéronique des composés de formule générale I a été déterminée selon le test de Clauberg modifié par Mac Phail (J. Physiol. (London) 83 (1934) 145).

Des lapines impubères d'un poids moyen de 1,5 kg ont reçu pendant 5 jours consécutifs une injection sous-cutanée de 5 mg de Benzoate d'Estradiol. Après ces cinq jours de sensibilisation, les animaux ont reçu à partir du sixième jour, tous les jours penant 5 jours, une injection par voie sous-cutanée du produit à tester ou de progestérone prise comme substance de comparaison. Les lapines sont sacrifiées après la dernière administration. Les utérus sont prélevés, fixés dans la solution de bouin puis inclus dans la Paraffine. On découpe au microtome des fragments de 5 µm de section que l'on colore avec le mélange Hemalun-Eosine-Safran. Le développement de la dentelle endométriale observée au microscope est déterminé selon l'échelle établie par Mac Phail.

Les composés de formule générale I ne provoquent pas de prolifération endométriale à des doses allant jusqu'à 25 mg pendant 5 jours. L'action pseudogestative est inférieure à 25% de celle de la progestérone.

L'action antiprogestéronique a été mise en évidence en utilisant une variante du test de Mac Phail. Après la phase préliminaire de sensibilisation par le benzoate d'estradiol, les animaux ont reçu par voie sous-cutanée une dose active de progestérone pendant 5 jours. Le composé de formule générale I est administré simultanément soit par voie sous-cutanée, soit par voie orale. 24 heures après la dernière administration, les animaux sont sacrifiés et les utérus sont analysés sous l'angle histologique. La prolifération endométriale a été déterminée selon l'indice de Mac Phail.

Résultats

a) après administration du composé de formule générale I par voie orale.

| Composé | Doses en mg/animal et par jour | Nombre d'animaux | Cotation selon l'indice Mac Phail |
|---|---|---|---|
| Progestérone | 0,24 mg | 7 | 2,9 ± 0,2 |
| Progestérone | 0,24 mg + composé | | |
| | de formule I 5 mg | 4 | 2,1 ± 0,7 (0,05≧p≧0,01) |
| | 10 mg | 4 | 0,6 ± 0,1 (p≦0,001) |
| | 20 mg | 4 | 0,8 ± 0,4 (p≦0,001) |
| | 40 mg | 4 | 0,1 ± 0,1 (p≦0,001) |
| Progestérone | 1,92 mg | 6 | 3,3 ± 0,1 |
| Progestérone | 1,92 mg + composé | | |
| | de formule I 5 mg | 4 | 3,3 ± 0,1 NS |
| | 10 mg | 3 | 3,0 ± 0,5 NS |
| | 20 mg | 4 | 0,8 ± 0,1 (p≦0,001) |
| | 40 mg | 4 | 0,9 ± 0,9 (p≦0,001) |

b) après administration sous-cutanée:

| Composé | Doses en mg/animal et par jour | Nombre d'animaux | Cotation selon l'indice Mac Phail |
|---|---|---|---|
| Progestérone | 0,24 mg | 7 | 2,9 ± 0,2 |
| Progestérone | 0,24 mg + composé | | |
| | de formule I 5 mg | 4 | 2,1 ± 0,7 (0,05≧p≧0,01) |
| | 10 mg | 4 | 0,6 ± 0,1 (p≦0,001) |
| | 20 mg | 4 | 0,8 ± 0,4 (p≦0,001) |
| | 40 mg | 4 | 0,1 ± 0,1 (p≦0,001) |
| Progestérone | 1,92 mg | 6 | 3,3 ± 0,1 |
| Progestérone | 1,92 mg + composé | | |
| | de formule I 5 mg | 4 | 3,3 ± 0,1 NS |
| | 10 mg | 3 | 3,0 ± 0,5 NS |
| | 20 mg | 4 | 0,8 ± 0,1 (p≦0,001) |
| | 40 mg | 4 | 0,9 ± 0,4 (p≦0,001) |

Les composés de formule générale I qui sont pratiquement dépourvus d'action progestomimétique, manifestent surtout par voie orale une action antiprogestéronique. Cette action est dépendante et proportionnelle à la dose administrée.

L'administration par voie sous-cutanée semble moins efficace.

**Revendications**

1oUtilisation d'un composé de formule générale I

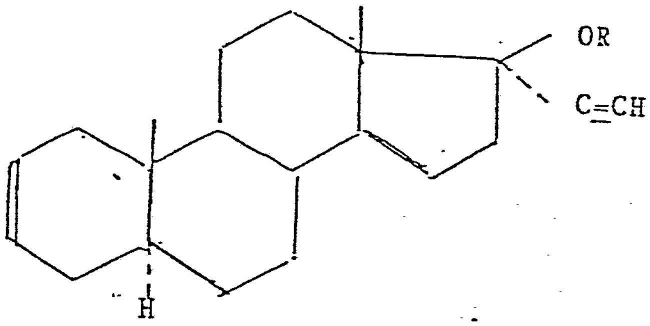

(I).

dans laquelle R est défini comme indiqué dans la description à des doses variant de 25 à 250 mg pour la réalisation d'un médicament utile pour supprimer les effets de la progestérone.

2o Utilisation d'un composé de formule générale I

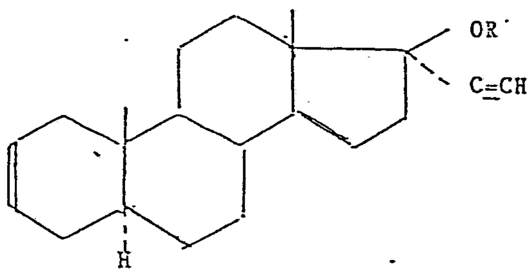

(I)

dans laquelle R est défini comme indiqué dans la description à des doses variant de 25 à 250 mg pour la réalisation d'un médicament utile pour empêcher la nidation.

3o Utilisation d'un composé de formule générale I

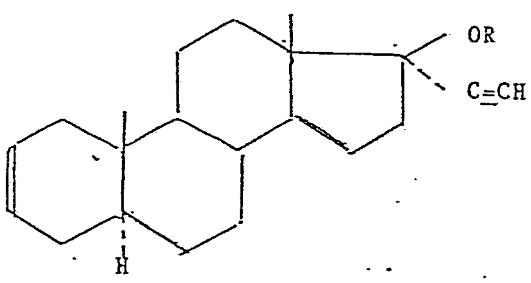

(I)

dans laquelle R est défini comme indiqué dans la description à des doses variant de 25 à 250 mg pour la réalisation d'un médicament utile pour obtenir l'interruption de gestation humaine

4o Procédé d'obtention d'un médicament ayant une action anti-progéstéronique selon la revendication 1 caractérisé en ce que l'on incorpore à un composé de formule générale I

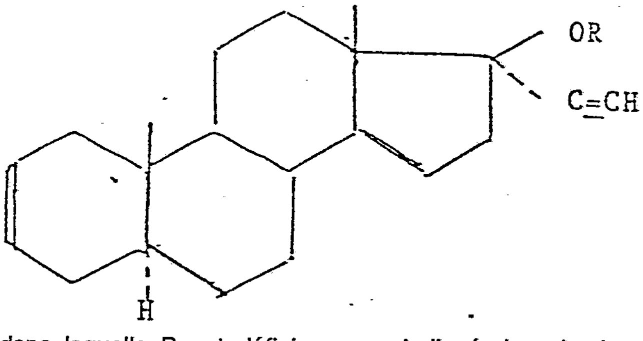

(I)

dans laquelle R est défini comme indiqué dans la description un excipient ou un véhicule inerte non-toxique pharmaceutiquement-acceptable,adapté à l'administration parentérale ,orale ou rectale.

5o Procédé d'obtention d'un médicament ayant une action anti-nidatoire selon la Revendication 2

caractérisé en ce que l'on incorpore à un composé de Formule générale I

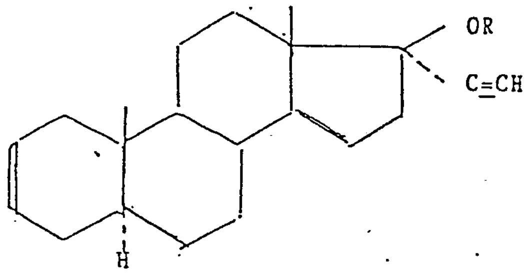

(I)

dans laquelle R est défini comme indiqué dans la description un excipient ou un vehicule inerte ,non-toxique ,pharmaceutiquement ment-acceptable ,adapté à l'administration parentérale ,orale ou recttale.

6o Procédé d'obtention d'un médicament à action abortive selon la Revendication 3 caractérisé en ce que l'on incorpore à un composé de Formule générale I

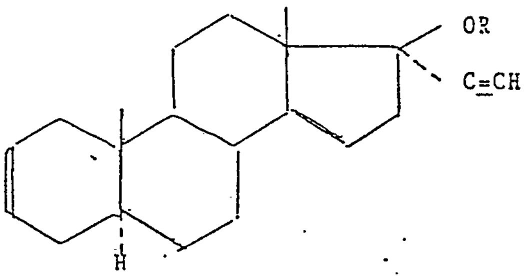

(I)

dans laquelle R est défini comme indiqué dans la description un excipient ou un vehicule inerte ,non-toxique pharmaceutiquement acceptable ,adapté à l'administration parentérale ,orale ou rectale.

Revendications pour les Etats contractants suivants: GRECE et ESPAGNE

1o Procédé d'obtention d'un médicament ayant une action anti-progéstéronique caractérisé en ce que l'on incorpore à un composé de formule générale I

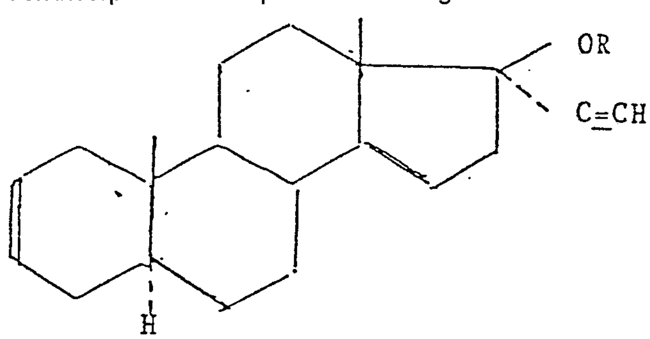

(I) .

dans laquelle R est défini comme indiqué dans la description un excipient ou un véhicule inerte non-toxique pharmaceutiquement-acceptable,adapté à l'administration parentérale ,orale ou rectale.

2o Procédé d'obtention d'un médicament ayant une action anti-nidatoire caractérisé en ce que l'on incorpore à un composé de Formule générale I

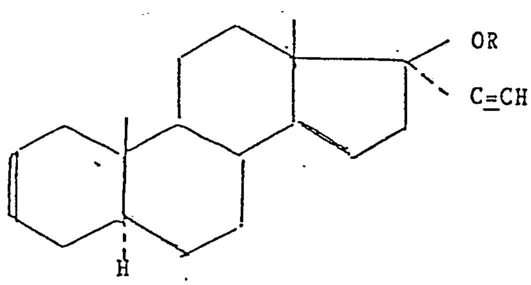

(I)

dans laquelle R est défini comme indiqué dans la description un excipient ou un vehicule inerte ,non-toxique ,pharmaceutiquement ment-acceptable ,adapté à l'administration parentérale ,orale ou rectatale.

3o Procédé d'obtention d'un médicament à action abortive caractérisé en ce que l'on incorpore à un

composé de Formule générale I

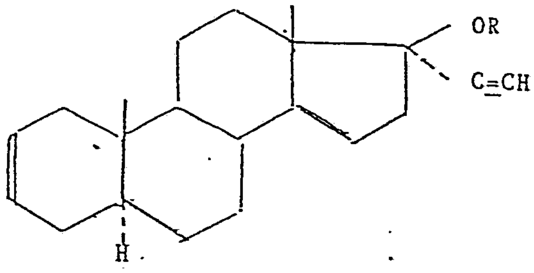

(I)

dans laquelle R est défini comme indiqué dans la description un excipient ou un vehicule inerte ,non-toxique pharmaceutiquement acceptable ,adapté à l'administration parentérale ,orale ou rectale.